# EUROPÄISCHE PATENTSCHRIFT

(11) **EP 1 281 358 B1**
(45) Veröffentlichungstag und Bekanntmachung des Hinweises auf die Patenterteilung: **06.10.2004**
(21) Anmeldenummer: 02017255.7
(22) Anmeldetag: 31.07.2002
(51) Int. Cl.: A61B 17/22, A61B 17/00

(54) **Vorrichtung zur Aufnahme und Halten eines Ureterostiums**
Device for receiving and holding the urethral ostium
Dispositif permettant la réception et le maintien d'un ostium urétral

(30) Priorität: 02.08.2001 DE 10137840
(43) Veröffentlichungstag der Anmeldung: 05.02.2003
(73) Patentinhaber: Urovision GmbH, 83043 Bad Aibling (DE)
(72) Erfinder: Dorschner, Wolfgang, 04299 Leipzig (DE)
(74) Vertreter: Hofstetter, Alfons J., Dr.rer.nat.

(56) Entgegenhaltungen:
- WO-A-94/21177
- US-A- 3 659 606
- US-A- 4 528 982

## Beschreibung

Die vorliegende Erfindung betrifft eine Vorrichtung zur Aufnahme und Halten eines Ureterostiums eines Menschen oder eines Säugetiers.

Der menschliche Ureter mündet in die Blase mit dem Ostium ureteris, einer physiologischen Enge, an der zum Beispiel Nierensteine hängen bleiben können. Auch sind Uretermündungsdefekte bekannt, die auf Fehlbildungen des Ureterostiums beruhen. Diese Defekte können zu Ureterschädigungen, Insuffizienz, Schrumpfblasen oder zu durch den Harnrückfluss bedingten Nierenschädigungen führen. Zudem treten Uretertumoren auf, zum Beispiel als Metastasen eines Blasentumors. In vielen Fällen ist es daher notwendig, dass der Ureter von der Blase im Bereich des Ureterostiums abgetrennt wird. Dabei tendiert der verbleibende Rest des Ureterostiumgewebes dazu, sich in die Blase hinein zu stülpen. Soll der verbleibende Ureterostiumrest nunmehr vernäht werden, so ist der Operateur gezwungen, diesen mit Hilfe einer chirurgischen Pinzette oder ähnlichem aus dem Blaseninneren herauszuholen. Erst dann ist es möglich, den Ureterostiumrest sicher zu vernähen.

Nachteiligerweise erschwert und verzögert diese übliche und bekannte Vorgehensweise den Operationsverlauf, was sich möglicherweise schädlich auf den Patienten auswirken kann.

Es ist daher Aufgabe der vorliegenden Erfindung eine Vorrichtung zur Aufnahme und Halten eines Ureterostiums eines Menschen oder eines Säugetiers bereitzustellen, welche eine ein einfaches und sicheres Halten des Ureterostiums gewährleistet.

Aus US 3,659,606 ist ein chirurgisches Instrument gemäß des Anspruchs 1 bekannt, das zum Herausziehen von Venen, insbesondere bei der Behandlung von Krampfaden verwendet wird. Dieses Instrument weist ein Kabel auf, das an beiden Enden mit zwei tubulären Plastikkappen verbunden ist. Nach Einführen dieser Instrumentes in die Vene kann auf das vordere Ende eine Spitze aufgesetzt werden. Das andere Ende des Instruments kann zur Aufnahme eines Griffs verwendet werden.

Gelöst wird diese Aufgabe durch eine Vorrichtung zur Aufnahme und Halten eines Ureterostiums eines Menschen oder eines Säugetiers mit den Merkmalen des Anspruchs 1.

Vorteilhafte Ausgestaltungen sind in den Unteransprüchen beschrieben.

Eine erfindungsgemäße Vorrichtung zur Aufnahme und Halten eines Ureterostiums eines Menschen oder eines Säugetiers umfasst einen Schaft, wobei an einem distalen Ende des Schafts ein Aufnahmeelement ausgebildet ist und das Aufnahmeelement eine Elementbasis mit einem größeren Durchmesser als der Schaft und eine sich in Vorschubrichtung der Vorrichtung verjüngende Elementspitze aufweist. Die erfindungsgemäße Vorrichtung kann in die durch das Abtrennen des Ureters von der Blase entstandene Öffnung in der Blase bzw. in den innerhalb der Blase liegenden Ureterostiumrest eingeführt werden. Die sich in Vorschubrichtung der Vorrichtung verjüngende Elementspitze des Aufnahmeelements gewährleistet ein problemloses und sicheres Einführen der Vorrichtung. Dadurch, dass die Elementbasis des Aufnahmeelements gegenüber dem Schaft der Vorrichtung verbreitert ausgebildet ist, ist es möglich, den Ureterostiumrest aufzunehmen, zu halten und durch eine Bewegung von der Blase weg aus dem Blaseninneren hervorzuholen. Anschließend kann das Ureterostium problemlos sicher und schnell vernäht werden. Dabei kann der Durchmesser des Basiselements bzw. des Aufnahmeelements im Bereich des Basiselements so gewählt werden, dass dieser größer ist als der Durchmesser des aufzunehmenden und zu haltenden Ureterostiums. Vorteilhafterweise beträgt der Durchmesser des Basiselements bzw. des Aufnahmeelements im Bereich des Basiselements zwischen 0,6-5,7 mm (11 und 17 Charr).

In einer vorteilhaften Ausgestaltung der Erfindung ist die Elementbasis des Aufnahmeelements zumindest teilweise kreisförmig oder ellipsoid ausgebildet und die Elementspitze ist zumindest teilweise abgerundet ausgebildet. Durch eine derartige Ausgestaltung des Aufnahmeelements ist ein sicheres Einführen der Vorrichtung in den Ureterostiumrest und ein entsprechend sicheres Herausführen dessen aus der Blase gewährleistet. Insbesondere werden durch diese abgerundeten Flächen Kanten vermieden, die zu Verletzungen des Ureterostiums führen können.

In einer weiteren vorteilhaften Ausgestaltung der Erfindung weist die Elementbasis des Aufnahmeelements mindestens einen zackenartigen Vorsprung auf. Dadurch ist es möglich Gewebeproben des Ureterostiums zu entnehmen und diese extrakorporal zu untersuchen. Dies ist insbesondere bei der chirurgischen Entfernung von Uretertumoren von Vorteil, da hiermit überprüft werden kann, ob sich noch Tumorreste im Bereich des Ureterostiumrests befinden.

In einer weiteren vorteilhaften Ausgestaltung der Erfindung ist an dem dem Aufnahmeelement gegenüberliegenden Ende des Schaftes ein Griffelement lösbar an dem Schaft angeordnet. Dadurch ist einerseits eine einfache Handhabung der erfindungsgemäßen Vorrichtung gewährleistet. Zudem erleichtert die Möglichkeit des Lösens des Griffelements vom Schaft die Probennahme von Geweberesten bzw. deren Lösen von der Vorrichtung.

Weitere Einzelheiten, Merkmale und Vorteile der Erfindung ergeben sich aus der nachfolgenden Beschreibung zweier in den Figuren dargestellten Ausführungsbeispiele. Es zeigen:
- Figur 1: eine schematische Darstellung einer erfindungsgemäßen Vorrichtung zur Aufnahme und Halten eines Ureterostiums eines Menschen oder eines Säugetiers;
- Figur 2: eine schematische Darstellung eines Aufnahmeelements einer erfindungsgemäßen Vorrichtung zur Aufnahme und Halten eines Ureterostiums gemäß einer ersten Ausführungsform;
- Figur 3: eine schematische Darstellung eines Aufnahmeelements einer erfindungsgemäßen Vorrichtung zur Aufnahme und Halten eines Ureterostiums gemäß einer zweiten Ausführungsform; und
- Figur 4: eine schematische Darstellung der Funktionsweise der erfindungsgemäßen Vorrichtung zur Aufnahme und Halten eines Ureterostiums mit einem Aufnahmeelement gemäß Figur 3.

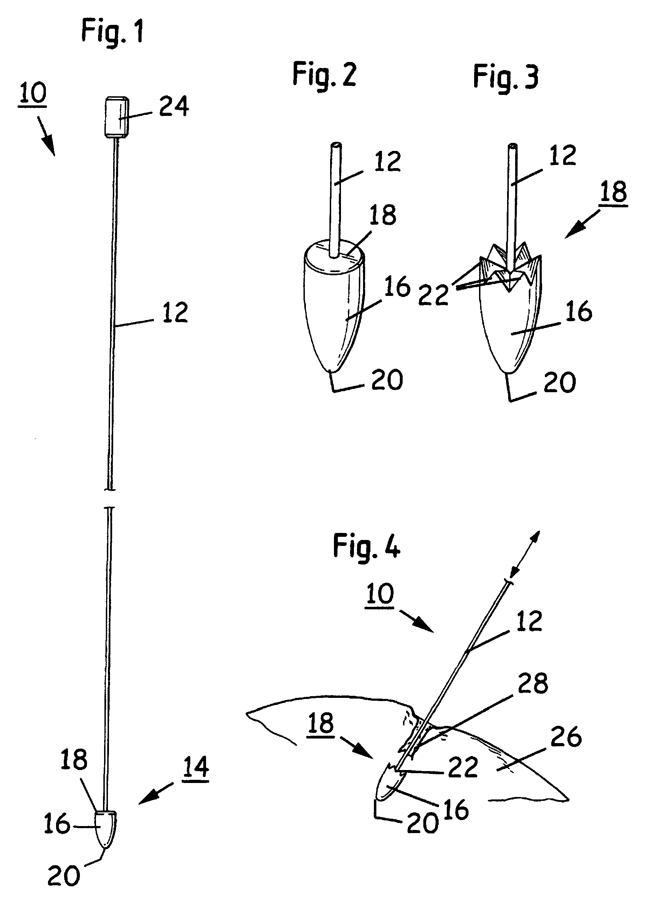

Figur 1 zeigt in einer schematischen Darstellung eine Vorrichtung 10 zur Aufnahme und Halten eines Ureterostiums eines Menschen oder eines Säugetiers mit einem Schaft 12. Der Schaft 12 weist einen kreisförmigen Durchmesser auf und kann flexibel oder starr ausgebildet sein. An einem distalen Ende 14 des Schafts 12 ist ein Aufnahmeelement 16 ausgebildet, wobei das Aufnahmeelement 16 eine Elementbasis 18 mit einem größeren Durchmesser als der Schaft 12 und eine sich in Vorschubrichtung der Vorrichtung 10 verjüngende Elementspitze 20 aufweist. Man erkennt, dass die Elementspitze 20 abgerundet als Halbrotationsellipsoid ausgebildet ist. Üblicherweise beträgt der Durchmesser des Basiselements 18 bzw. des Aufnahmeelements 16 im Bereich des Basiselements 18 zwischen 0,6-5,7 mm (11 und 17 Charr).

An dem dem Aufnahmeelement 16 gegenüberliegenden Ende des Schaftes 12 ist ein Griffelement 24 lösbar an dem Schaft 12 angeordnet. Das Griffelement 24 ist dabei zylindrisch ausgebildet. Es ist jedoch möglich, dass das Griffelement 24 zusätzliche Griffmulden (nicht dargestellt) zur Vereinfachung der Handhabung aufweist.

Der Schaft 12 und/oder das Aufnahmeelement 16 und/oder das Griffelement 24 bestehen aus Metall und/oder einer Metall-Legierung und/oder Kunststoff und/oder Silikon.

Figur 2 zeigt in einer schematischen Darstellung ein Aufnahmeelement 16 der Vorrichtung 10 gemäß einer ersten Ausführungsform, wie sie auch in Figur 1 dargestellt worden ist. Man erkennt, dass die Elementbasis 18 kreisförmig ausgebildet ist. Es ist aber auch möglich, dass die Elementbasis 18 eine elliptische Form aufweist. Des weiteren erkennt man, dass die Elementspitze 20 als Halbrotationsellipsoid ausgebildet ist. Es ist aber auch möglich, dass die Elementspitze 20 eine Halbkugel-Form aufweist. Das Aufnahmeelement 16 ist einstückig mit dem Schaft 12 verbunden. Auch eine lösbare Verbindung zwischen dem Schaft 12 und dem Aufnahmeelement 16 ist denkbar.

Figur 3 zeigt in einer schematischen Darstellung ein Aufnahmeelement 16 der Vorrichtung 10 gemäß einer zweiten Ausführungsform. Man erkennt, dass auch hier die Elementbasis 18 kreisförmig und die Elementspitze 20 als Halbrotationsellipsoid ausgebildet ist. Die Elementbasis 18 weist jedoch zusätzlich mehrere zackenartige Vorsprünge 22 auf. Die Vorsprünge 22 sind dabei rotationssymmetrisch am Umfang des kreisförmigen Basiselements 18 verteilt.

Figur 4 zeigt in einer schematischen Darstellung die Funktionsweise der Vorrichtung 10 mit einem Aufnahmeelement 16 gemäß Figur 3. Man erkennt, dass die Vorrichtung 10 in die durch das Abtrennen des Ureters von der Blase 26 entstandene Öffnung in der Blase 26 bzw. in den innerhalb der Blase 26 liegenden Ureterostiumrest 28 eingeführt wird. Die sich in Vorschubrichtung der Vorrichtung 10 verjüngende Elementspitze 20 des Aufnahmeelements 16 gewährleistet ein problemloses und sicheres Einführen der Vorrichtung 10. Dadurch, dass die Elementbasis 18 des Aufnahmeelements 16 gegenüber dem Schaft 12 der Vorrichtung 10 verbreitert ausgebildet ist, ist es möglich, den Ureterostiumrest 28 aufzunehmen, zu halten und durch eine Bewegung von der Blase 26 weg aus dem Blaseninneren hervorzuholen. Anschließend kann das Ureterostium 28 problemlos sicher und schnell vernäht werden. Dabei kann der Durchmesser des Basiselements 18 bzw. des Aufnahmeelements 16 im Bereich des Basiselements 18 so gewählt werden, dass dieser größer ist als der Durchmesser des aufzunehmenden und zu haltenden Ureterostiums 28.

## Patentansprüche

1. Vorrichtung zur Aufnahme und zum Halten eines Ureterostiums eines Menschen oder eines Säugetiers, mit einem Schaft (12) zum Einführen eines Aufnahmeelements (16) in einen Ureterostiumrest, wobei das Aufnahmeelement (16) an einem distalen Ende (14) des Schafts (12) ausgebildet ist und einer Elementbasis (18) mit einem größeren Durchmesser als der Schaft (12) sowie einer sich in Vorschubrichtung der Vorrichtung (10) ausgehend von der Elementbasis verjüngenden Elementspitze (20) zugeordnet ist, wobei die Elementbasis (18) eine im wesentlichen elliptische Grundfläche aufweist, wobei auf der Grundfläche mindestens ein zackenartiger Vorsprung (22) angeordnet ist.

2. Vorrichtung nach Anspruch 1,
**dadurch gekennzeichnet,**
**dass** die Elementspitze (20) zumindest teilweise abgerundet ausgebildet ist.

3. Vorrichtung nach Anspruch 2,
**dadurch gekennzeichnet,**
**dass** die Elementspitze (20) als Halbkugel oder Halbrotationsellipsoid ausgebildet ist.

4. Vorrichtung nach einem der vorhergehenden Ansprüche,
**dadurch gekennzeichnet,**
**dass** der Durchmesser des Basiselements (18) bzw. des Aufnahmeelements (16) im Bereich des Basiselements (18) zwischen 0,6-5,7 mm (11 und 17 Charr) beträgt.

5. Vorrichtung nach einem der vorhergehenden Ansprüche,
**dadurch gekennzeichnet,**
**dass** an dem dem Aufnahmeelement (16) gegenüberliegenden Ende des Schaftes (12) ein Griffelement (24) lösbar an dem Schaft (12) angeordnet ist.

6. Vorrichtung nach einem der vorhergehenden Ansprüche,
**dadurch gekennzeichnet,**
**dass** das Aufnahmeelement (16) lösbar oder einstückig mit dem Schaft (12) verbunden/ausgebildet ist.

7. Vorrichtung nach einem der vorhergehenden Ansprüche,
**dadurch gekennzeichnet,**
**dass** der Schaft (12) und/oder das Aufnahmeelement (16) und/oder das Griffelement (24) aus Metall und /oder einer Metall-Legierung und/oder Kunststoff und/oder Silikon besteht/bestehen.

8. Vorrichtung nach einem der vorhergehenden Ansprüche,
**dadurch gekennzeichnet,**
**dass** auf der Grundfläche am Umfang der Elementbasis (18) mehrere zackenartige Vorsprünge (22) rotationssymmetrisch angeordnet sind.

## Claims

1. Device for receiving and for retaining an ureterostium of a human or of a mammal, including a shaft (12) for inserting a receiving member (16) into an ureterostium residual, wherein the receiving member (16) is formed at a distal end (14) of the shaft (12) and is associated with a member base (18) having a greater diameter than the shaft (12), as well as with a member tip (20) tapering in advance direction of the device (10) starting from the member base, wherein the member base (18) has a substantially elliptical base surface, wherein at least one prong-like projection (22) is disposed on the base surface.

2. Device according to claim 1,
**characterized in that**
the member tip (20) is formed at least partially rounded.

3. Device according to claim 2,
**characterized in that**
the member tip (20) is formed as a hemisphere or semi-ellipsoid of revolution.

4. Device according to any one of the preceding claims,
**characterized in that**
the diameter of the base member (18) or of the receiving member (16) in the region of the base member (18), respectively, is between 0.6-5.7 mm (11 and 17 Charr).

5. Device according to any one of the preceding claims,
**characterized in that**
at the end of the shaft (12) opposing the receiving member (16), a grip member (24) is disposed detachably on the shaft (12).

6. Device according to any one of the preceding claims,
**characterized in that**
the receiving member (16) is connected/formed detachably or integrally to/with the shaft (12).

7. Device according to any one of the preceding claims,
**characterized in that**
the shaft (12) and/or the receiving member (16) and/or the grip member (24) is/are made of metal and/or a metal alloy and/or plastics and/or silicone.

8. Device according to any one of the preceding claims,
**characterized in that**
on the base surface, on the circumference of the member base (18), plural prong-like projections (22) are disposed rotational-symmetrically.

## Revendications

1. Dispositif de réception et de maintien d'un urétérostium d'un être humain ou d'un mammifère, comprenant une tige (12) d'introduction d'un élément de prélèvement (16) dans un reste d'urétérostium, l'élément de prélèvement (16) étant conformé à une extrémité distale (14) de la tige (12), tout en étant associé à une base d'élément (18) présentant un diamètre excédant celui de la tige (12) ainsi qu'à une pointe d'élément (20) s'amincissant en direction d'avance du dispositif (10), s'étendant de la base de l'élément, la base d'élément (18) présentant une surface de base sensiblement elliptique, au moins une projection sous forme de dent (22) étant prévue sur la surface de base.

2. Dispositif selon la revendication 1,
**caractérisé en ce que**
la pointe de l'élément (20) est prévue, au moins en partie, sous forme arrondie.

3. Dispositif selon la revendication 2,
**caractérisé en ce que**
la pointe de l'élément (20) est prévue sous forme d'une hémisphère ou d'un ellipsoïde à demi-rotation.

4. Dispositif selon l'une des revendications précédentes,
**caractérisé en ce que**
le diamètre de l'élément de base (18) resp. de l'élément de prélèvement (16) est compris, au droit de l'élément de base (18), entre 0,6 et 5,7 mm (11 et 17 Charr).

5. Dispositif selon l'une des revendications précédentes,
**caractérisé en ce qu'**à
l'extrémité de la tige (12) opposée à l'élément de prélèvement (16), un élément de préhension (24) est rapporté à la tige (12) de façon amovible.

6. Dispositif selon l'une des revendications précédentes,
**caractérisé en ce que**
l'élément de prélèvement (16) est soit associé de manière amovible à la tige (12) soit solidaire de celle-ci.

7. Dispositif selon l'une des revendications précédentes,
**caractérisé en ce que**
la tige (12) et/ou l'élément de prélèvement (16) et/ou l'élément de préhension (24) est réalisée/sont réalisés en métal et/ou en un alliage métallique et/ou en une matière plastique et/ou en silicone.

8. Dispositif selon l'une des revendications précédentes,
**caractérisé en ce que**
sur la surface de base, à la périphérie de la base d'élément (18), plusieurs projections (22) sous forme de dents sont disposées à symétrie de rotation.
